# EUROPEAN PATENT APPLICATION

(11) **EP 4 101 367 A1**
(43) Date of publication of application: **14.12.2022**
(21) Application number: 21178609.0
(22) Date of filing: 09.06.2021
(51) Int. Cl.: A61B 3/02, A61B 3/024, A61B 3/028, A61B 3/06, A61B 3/103

(54) **METHOD AND DEVICE FOR DETERMINING A VISUAL PERFORMANCE**

(71) Applicant: Carl Zeiss Vision International GmbH, 73430 Aalen (DE)
(72) Inventor: Leube, Alexander, 73434 Aalen (DE); Nehrbass, Eric, 73431 Aalen (DE); Urban, Steffen, 07749 Jena (DE); Achilles, Christian, 07745 Jena (DE); Weimann, Claudius, 73560 Böbingen (DE)
(74) Representative: Altmann Stößel Dick Patentanwälte PartG mbB

(57) **Abstract**

The present invention relates to a computer-implemented method (210), a computer program and a device (110) for determining a visual performance of at least one eye (112) of a person (114) as well as a method for producing at least one spectacle lens (228). Herein, the method (210) comprises the following steps:
a) identifying (212) a point in time indicating that a person (114) is able to perceive at least one visual target (128) from a reaction of the person (114);
b) determining (216) a visual performance (218) of at least one eye (112) of the person (114) by using at least one first piece of information about the at least one visual target (128) and at least one second piece of information about at least one parameter (168) related to the at least one visual target (128) at the point in time,
c) recognizing (214) the at least one visual target (128) and the at least one parameter (168) related to the at least one visual target (128) at the point in time by scanning a natural environment (122) of the person (114).

## Description

### Field of the invention

The present invention relates to a computer-implemented method, a computer program and a device for determining a visual performance of at least one eye of a person as well as a method for producing at least one spectacle lens. Determining the visual performance, specifically at least one of contrast sensitivity, visual acuity, color vision or visual field, of the at least one eye of the person can, in particular, be used as examination method for at least one of a detection of at least one visual impairment, in particular for determining at least one refractive error of at least one eye of a person, such as myopia, hyperopia or astigmatism, or an observation of a treatment success of the at least one visual impairment. However, further applications may also be feasible.

### Related art

Assessing visual performance, specifically at least one of contrast sensitivity, visual acuity, color vision or visual field, of one or both eyes of a person requires a standardized testing procedure comprising a clear relationship between a testing distance and visual target properties, in particular, selected from at least one of size, contrast, or color, wherein the standardized testing procedure, generally, uses expensive devices and expert knowledge.

Current methods and devices for determining a visual performance of at least one eye of a person still utilize an acuity board in order to display standard vision tests to person. As an alternative, a mobile communication device, such as a smartphone, a tablet, or a laptop, can be used to display standard vision tests for basics testing at home, however, they still require further equipment, especially both an external screen configured to display the visual targets and an input device configured to be synchronized with the external screen.

Further, methods and devices for mapping RGB camera information combined with depth information in order to acquire RGBD images and to segment objects in natural environments are already known, wherein the term "RGB" refers to a red, green, and blue color space, wherein the term "RGBD" indicates that the color space is extended by the depth information.

US 2016/0379074 A1 discloses a method and a system for tracking mobile objects in a site. The system comprises a computer cloud communicating with one or more imaging devices and one or more tag devices. Each tag device is attached to a mobile object, and has one or more sensors for sensing the motion of the mobile object. The computer cloud visually tracks mobile objects in the site using image streams captured by the imaging devices, and uses measurements obtained from tag devices to resolve ambiguity occurred in mobile object tracking. The computer cloud uses an optimization method to reduce power consumption of tag devices.

US 10,130,548 B2 discloses a lighting device which obtains data related to objects and boundaries in an area in a vicinity of the lighting device, and a user wearable device which provides a display (e.g. an augmented reality display based on the data related to the objects and the area boundaries) for a user/wearer. The lighting device includes a mapping sensor that collects data related to the objects and boundaries in the area. The user wearable device includes a camera or other optical sensor and wireless communication capability. The user wearable device is provided with mapping data that is presented on a display of the user wearable device. The communications and display capabilities allow the user wearable device to obtain room mapping information related to area in the vicinity of the lighting device in order to provide navigational assistance to a visually impaired person in the area.

US 10,251,545 B2 discloses a process for assessing at least one characteristic of a user's vision including determining a characteristic of at least one camera of a mobile device; using the at least one camera, capturing at least one image of an object; determining, with reference to a portion of the at least one image composed of the object, a distance from the mobile device to the object at a time of capture of the at least one image; and receiving input from the user in response to material presented on the object.

US 10,314,475 B2 discloses a server in communication with a first device capable of receiving input from a camera attached to the first device, and a second device capable of displaying output. The server is configured to receive, from the first device, data relating to characteristics of the camera and characteristics of an image obtained by the camera of a screen of the second device; determine a screen pixel length of the screen; and provide instructions to the second device to display on the screen an object at a desired visual angle in response to the data received from the second device and the screen pixel length, while the first device is positioned at an arbitrary distance from the screen.

Tian et al., Toward a computer vision-based wayfinding aid for blind persons to access unfamiliar indoor environments, Machine Vision and Applications 24, 2013, pp. 521-535, propose a proof-of-concept computer vision-based wayfinding aid for blind people to independently access unfamiliar indoor environments. In order to find different rooms and other building amenities, object detection is incorporated with text recognition. First, a robust and efficient algorithm is developed to detect doors, elevators, and cabinets based on their general geometric shape, by combining edges and corners. The algorithm is general enough to handle large intraclass variations of objects with different appearances among different indoor environments, as well as small inter-class differences between different objects such as doors and doorlike cabinets. Next, to distinguish intra-class objects, e.g. an office door from a bathroom door, text information associated with the detected objects is extracted and recognized. For text recognition, text regions from signs with multiple colors and possibly complex backgrounds are, firstly, extracted and character localization and topological analysis to filter out background interference are, subsequently, applied. The extracted text is recognized using off-the-shelf optical character recognition software products. The object type, orientation, location, and text information are presented to the blind traveler as speech.

Further, VeyeZER™: Visual Assessments, accessible via veyezer.com (retrieved April 13, 2021), presents the VeyeZER^{™} application which leverages Augmented Reality on the Microsoft Hololens 1 headset to modernize the practice of visual assessments. Using IP processes, VeyeZER^{™} accommodates both refraction and binocular testing, including vergence and duction, with just a headset and a laptop. This method of testing is completely portable, meaning testing can be brought to the patient. No more limitations are imposed by office environments and a need of large static testing equipment.

WO 2020/216789 A1 discloses a method, a device, and a computer program for determining a refractive error of at least one eye of a user, and a method for manufacturing a spectacle lens for the at least one eye of the user. The method for determining the refractive error of the at least one eye of the user comprises the following steps: a) displaying at least one character on a screen, wherein the at least one character displayed on the screen is at least one periodic pattern, wherein at least one parameter of the at least one pattern displayed on the screen comprises at least one spatial frequency, wherein the at least one parameter of the at least one character displayed on the screen is varied; b) detecting a reaction of the user depending on the at least one character displayed on the screen; c) determining a point in time at which the reaction of the user results in the user being able to perceive the at least one character displayed on the screen; and d) determining a value for the refractive error of the at least one eye of the user from the at least one parameter determined at the point in time, wherein the value for the refractive error is determined from the at least one spatial frequency, determined at the point in time, of the at least one pattern.

### Problem to be solved

In particular with respect to the disclosure of WO 2020/216789 A1, it is, therefore, an objective of the present invention to provide a computer-implemented method, a computer program and a device for determining a visual performance of at least one eye of a person as well as a method for producing at least one spectacle lens, which at least partially overcome the above-mentioned problems of the state of the art.

It is a particular objective of the present invention to assess the visual performance one or both eyes of a person by using a virtual reality headset, an augmented reality overlay or a mobile communication device, specifically a smartphone, a tablet, or a laptop, without requiring any further equipment, such as an external screen configured to display the visual targets and an input device configured to be synchronized with the external screen, to allow determining the visual performance at any place by any person without using an expensive device nor expert knowledge.

### Summary of the invention

This problem is solved by a computer-implemented method, a computer program and a device for determining a visual performance of at least one eye of a person as well as a method for producing at least one spectacle lens having the features of the independent claims. Preferred embodiments, which can be implemented in an isolated fashion or in any arbitrary combination, are listed in the dependent claims and throughout the specification.

In a first aspect, the present invention relates to a computer-implemented method for determining a visual performance of at least one eye of a person. As generally used herein, the term "at least one eye" refers to a single eye of the person or to both eyes of the person. The method for determining a visual performance of at least one eye of a person comprises the following steps a) to c):
a) identifying a point in time indicating that a person is able to perceive at least one visual target from a reaction of the person;
b) determining a visual performance of at least one eye of the person by using at least one first piece of information about the at least one visual target and at least one second piece of information about at least one parameter related to the at least one visual target at the point in time; and
c) recognizing the at least one visual target and the at least one parameter related to the at least one visual target at the point in time by scanning a natural environment of the person.

Herein, the indicated steps a) to c) may, preferably, be performed in an order commencing with step a) and continuing with step c), or commencing with step c) and continuing with step a), and finishing with step b). However, any or all of the indicated steps, in particular steps a) and c), may performed concurrently at least in part and/or be repeated several times. Further steps, whether disclosed herein or not, may, in addition, be performed in the context of the present method.

As already indicated, the method according to the present invention is a computer-implemented method. As generally used, the term "computer-implemented method" refers to a method which involves a programmable apparatus, in particular a processing unit, specifically a computer, such as a stationary computer, a computer network, or a readable medium carrying a computer program, whereby at least one feature of the method is performed using the at least one computer program. Alternatively, the at least one computer program may be accessible by an apparatus configured to perform the method via at least one of:
- a network, such as an in-house network or the internet;
- a virtual reality device, such as a virtual reality headset;
- an augmented reality device, such as an augmented reality (AR) overlay; or
- a mobile communication device.

As generally used, the term "mobile communication device" refers to a smartphone, a tablet, or a laptop, which can be carried by the person and, can thus, move together with the person. However, further kinds of mobile communication devices may also be conceivable. Herein, the at least one mobile communication device may comprise at least one operating system that may be configured to facilitate a use of software, multimedia functionalities, and communication facilities, especially an access to internet or to at least one wireless communication protocol, such as Wi-Fi or Bluetooth.

As indicated above, the present method related to determining the visual performance of at least one eye of a person. As generally used, the term "determining" or any grammatical variation thereof relates to a process of generating at least one representative result, in particular a plurality of representative results, with respect to the visual performance of the at least one eye of a person by applying the method as disclosed herein.

As further used herein, the term "visual performance" refers to a property of at least one eye of a person which can be determined by investigating the at least one eye of a person by an adapted measurement procedure. In particular, the visual performance can be selected from at least one property of the at least one eye of the person, in particular:
- a contrast sensitivity;
- a visual acuity;
- a color vision; or
- a visual field
of the at least one eye of the person. As generally used, the term "contrast sensitivity" refers to a property of at least one eye of a person to discern between different luminance levels in at least one visual target. As further generally used, the term "visual acuity" refers to a spatial resolution ability of the at least one eye of the person with respect to a structure within at least one visual target. As further generally used, the term "color vision" refers to a property of the at least one eye of the person to discern between different colors comprised by at least one visual target. As further generally used, the term "visual field" refers to a spatial array that can be viewed by the at least one eye of the person, in particular, when intending to gaze the view on a particular item, such as a fixation cross. Furthermore, each property of the at least one eye of the person can, alternatively or in addition, be determined in at least one of a static fashion or a dynamic fashion. As used in this regard, the term "static" refers to at least one single value of the respective property at a particular point in time, while the term "dynamic" refers to a plurality of values representing a temporal course of the respective property.

In addition, at least one further property of the at least one eye of the person may be determined by using the visual performance of the at least one eye of the person as determined in accordance with the method as disclosed herein, specifically at least one refractive error of the at least one eye of the person from the corresponding contrast sensitivity. Herein, the method for determining the visual performance of the at least one eye of the person may, further, comprise step j) as follows:
j) determining at least one refractive error of the at least one eye of the person by using the visual performance of the at least one eye of the person.

As generally used, the terms "refraction" or "refractive" refer to a bending of incident light entering the interior of the eye of the person via the pupil, wherein the term "refractive error" refers to an observation that the incident light may, in particular owing to a form of the eye, not be focusing appropriately on the retina of the eye, resulting in a defocus of the eye. In particular, the contrast sensitivity as a function of spatial frequency follows a characteristic curve, wherein a shape of the curve, especially a slope of the curve, changes with the defocus of the person. From measurements of the contrast sensitivity for at least two different spatial frequencies, the shape of the contrast sensitivity function can be associated with the defocus, i.e. with the refractive error of the eye.

According to step a), a point in time indicating that a person is able to perceive at least one visual target from a reaction of the person is identified. As generally used, the term "identifying" or any grammatical variation thereof relates to a process of generating at least one numeric value, in particular exactly one numerical value, which is related to a specified object or event. Herein, the numerical value refers to a point in time, wherein the term "point in time" specifies a particular moment in which a particular reaction of the person indicates that the person is able to perceive the at least one visual target. As further used herein, the term "perceiving" or any grammatical variation thereof indicates that the person is capable of recognizing the at least one visual target.

As generally used, the term "visual target" refers to at least one of a two-dimensional representation or a three-dimensional representation of at least one of an object or an abstract idea. In particular, the at least one visual target may be selected from a number, a letter, or a symbol, which may be suitable for being perceived by the person. By way of example, the visual target may comprise or be similar to such a kind of number, letter, or symbol which has, typically, been used in known standard vision tests that are familiar to the person skilled in the art over a long period of time. However, a further kind of visual target may also be feasible. In general, the visual target may be selected from at least one of static target or a dynamic target. As used in this regard, the term "static" refers to a kind of visual target which remains unamended over a period of time, preferably, as long as the person may view at this kind of visual target. In contrast hereto, the term "dynamic" refers to a further kind of visual target which changes in a time-varying manner over a period of time, in particular in a fashion that a least a part of this kind of visual target varies its appearance during it may be viewed by the person.

Further, each visual target has the at least one parameter which is related to the at least one visual target, wherein the term "parameter" refers to a property of the at least one visual target which can, specifically, be expressed by using at least one of a numerical value or an alphanumerical value. Preferably, the at least one parameter may be selected from at least one of a size or an angular size of the at least one visual target, a contrast or a contrast gradient of the at least one visual target, a color or a color gradient of the at least one visual target, a spatial position or a velocity of a movement of the at least one visual target, or a distance of the at least one visual target to the person. Herein, the term "size" refers to an extension of the at least one visual target, whether indicated by a length or by a viewing angle. Further, the term "contrast" relates to a luminance level in the at least one visual target. Further, the term "color" indicates a wavelength of the at least one visual target. Further, the term "spatial position" refers to a location of the at least one visual target. Further, the term "velocity of movement" refers to a velocity by the at least one visual target changes its position. Further, the term "distance" relates to a spacing between the at least one visual target and at least one eye of the person. However, at least one further parameter which may be related to the at least one visual target may be conceivable.

In a particularly preferred embodiment, identifying the point in time which indicates that the person is capable of perceiving the at least one visual target may comprise recording the reaction of the person to the at least one visual target. As generally used, the term "reaction" refers to response of the person which is provided by the person upon perceiving or not perceiving the at least one visual target. As generally used, the term "recording" relates to any kind of determining the reaction of the person, either by observing a behavior of the person or, alternatively or in addition, by monitoring at least one measurement signal, especially at least one electronic signal, which can be provided by at last one input unit configured to generate the at least one measurement signal upon occurrence of the reaction of the person. Herein, the reaction of the person to the at least one visual target can be received in form of a touch response by generating the at least one measurement signal upon occurrence of a contact of the person with the at least one input unit, in particular when the person is touching the touch-sensitive screen of a mobile communication device. Alternatively or in addition, the reaction of the person to the at least one visual target can be received in form of a voice entry by generating the at least one measurement signal upon receiving of at least one acoustic signal, in particular by a microphone as comprised by a mobile communication device. However, generating the at least one measurement signal in a different fashion may also be feasible. The measuring signal can be provided to a processing unit as described below in more detail, which is configured to identify the point in time that indicates that the reaction of the person relates to the perceiving of the at least one visual target by the person.

Alternatively or in addition, the reaction of the person to the at least one visual target can be recorded by observing the behavior of the person. Herein, the reaction of the person to the at least one visual target can be determined by gesture recognition, in particular by using at least one algorithm which is configured to identify a reaction of the person from a non-verbal expression of the person, specifically from an alteration in the face of the person that can be interpreted as the desired reaction of the person. As described below in more detail, at least one irregular movement in the face of the person can be detected for such a purpose, wherein the at least one irregular movement may indicate a visual strain of the at least one eye of the person related to perceiving the at least one visual target.

In a preferred embodiment, at least one invitation can be forwarded to the person in order to support and/or to prompt the person to provide the expected reaction in a desired fashion. As generally used, the term "invitation" refers to at least one of a request, such as an instruction, which asks the person to perform a particular task, or to a proposal, such as a suggestion, which provides further information to the person, specifically, for improving a performance of a particular task. Herein, the invitation can be provided to the person by using a voice output, in particular a loudspeaker, which may be configured to forward the appropriate commands to the person in an acoustic fashion. Alternatively or in addition, the invitation can be provided to the person by using a visual output, in particular a screen, which may be configured to forward the appropriate commands to the person in a visual fashion, specifically in form of a text message. However, further alternatives such as a tactile output, may also be feasible. In a particular embodiment, the invitation can be generated by at least one processing unit, in especially, by using at least one algorithm, such as a bot. As generally used, the term "bot" relates to at least one software application which is configured to perform at least one particular task in an automatic fashion, specifically via a network, such as the internet. In particular, the reaction of the person can be recorded simultaneously for both eyes or, alternatively or in addition, in a consecutive fashion for each eye. For recording the reaction of the person in a consecutive fashion, one of the eyes of the person can be covered, in particular initiated by a corresponding invitation.

In general, identifying the point in time which indicates that the person is capable of perceiving the at least one visual target can be performed in real time. However, according to the present invention, identifying the point in time which indicates that the person is capable of perceiving the at least one visual target can, alternatively or in addition, be performed at a later time and, preferably, in an off-line fashion.

In a further preferred embodiment, identifying the point in time which indicates that the person is capable of perceiving the at least one visual target can be improved by performing at least one training of the process of recording the reaction of the person to the at least one visual target. As generally used, the term "training" indicates that a performance of a particular process is improved during a training phase by providing a plurality of training data sets and executing them in the particular process. Herein each training data set which is used for a training purpose may, in particular, comprise an electronic representation of the reaction of the person, such as in form of at least one of a touch response, a voice entry, a gesture, or a type of eye movements elicited in the at least one eye of the person, wherein the at least one eye of the person has a known visual performance, to a known visual target having at least one known parameter related to the at least one visual target. The particular process is, then, performed with the particular training data set, wherein the result in form of the reaction of the person as observed in this fashion is adjusted to the known visual performance of the at least one eye of the person, the known visual target and the at least one known parameter related to the at least one visual target of the particular training data set.

Herein, a plurality of training data sets is iteratively applied during the training phase in order to improve an approximation of the result as achieved during the execution of the particular process, specifically by repeated the training of the particular process until a deviation between the reaction of the person as obtained by executing the training process, the known visual target and the at least one known parameter related to the at least one visual target as comprised by each training data set may be below a threshold. After the training phase, the reaction of the person as obtained by executing the training process can reasonably be expected to approximate the known visual target having the at least one known parameter related to the at least one visual target by the person having the visual performance in the same manner as achieved during the training phase. In this fashion, a more accurate determination of the at least one reaction of the person to the visual target can be obtained during the training phase. Thus, after the training phase, the desired performance of the particular process may be acquired.

The identifying of the point in time which indicates that the person is capable of perceiving the at least one visual target may be performed in accordance with a predefined scheme, however, artificial intelligence, in particular machine learning, may also be applied, especially by using a neuronal network. As generally used, the term "machine learning" refers to a process of applying artificial intelligence to automatically generate a statistical model for classification or regression. A machine learning algorithm configured to generate the desired model based on a large number of training data sets can, preferably, be used. Herein, the machine learning algorithm can be a supervised algorithm or a self-learning algorithm. The machine learning algorithm can use and/or comprise a neural network, which may, preferably, be developed into a trained neural network by using the at least one training data set. The neural network may comprise at least one element selected from hierarchical decision trees, Hough forest, regression forest, Convolutional Neural Network (CNN), Deep Neural Network (DNN) Residual Neural Network, Pixel-wise Voting, Pixel-wise Fusion Network, Deep learning. Alternatively or additionally, the use of at least one other artificial intelligence method, preferably a kernel method, especially a Support Vector Machine (SVM), may also be feasible.

According to step c), the at least one visual target and the at least one parameter related to the at least one visual target at the point in time are recognized by scanning a natural environment of the person. In contrast to known computer-implemented methods for determining the visual performance of at least one eye of a person, wherein the at least one visual target is generated on a screen by using a processing unit being configured to select the at least one visual target and to determine the at least one parameter related to the at least one visual target in a fashion to be presented to the person, the computer-implemented method for determining the visual performance of at least one eye of a person according to the present invention uses at least one visual target having at least one related parameter which is already available as is in the natural environment of the person, wherein both the at least one visual target and the at least one parameter related to the at least one visual target may be recognized by scanning the natural environment of the person.

As used herein, the term "environment" refers to a three-dimensional space which at least partially, i.e. partially or fully, surrounds the person as well as a device which is used for determining the visual performance of the at least one eye of a person as described below in more detail. In particular, the space surrounding the person and the device may be a room or a portion thereof, in particular a personal room in an accommodation at which the person may reside, a room in a shop of optician, or a room in a practice of an ophthalmologist; however, further kinds of rooms may also be feasible. In practice, using an indoor environment may be preferred, in particular since the indoor environment may, generally, comprise a larger number of visual targets and may facilitate maintaining a constant luminance level. However, using an outdoor environment may also be feasible.

As further used herein, the term "natural" indicates that the room or the portion thereof is used as is, wherein the term "as is" indicated that no prior preparation the room or the portion thereof is required for the purposes of the present invention. In addition, the term "natural" may also include that at least one of the visual targets may be rearranged by the person within the room or the portion thereof, such as by at least one of moving the at least one of the visual targets to a different location within the room or the portion thereof, or by altering an orientation the at least one of the visual targets. Herein, rearranging the at least one of the visual targets may, preferably, be triggered by at least one corresponding invitation to the person or an assistant. In addition, the term "natural" may, further, include that at least one further visual target may, additionally, be placed in the room or the portion thereof, in particular in an event in which the room or the portion thereof as is may not comprise a sufficient number of visual targets which may be suitable for being perceived by the person according to the present invention. In this event, the invitation that can, preferably, be forwarded to the person may comprise initiating the person or any other person, in particular an eye care professional, such as an optician, an ophthalmologist or an assistant, to place at least one further visual target within the room or the portion thereof in a desired fashion. As already described above, the invitation can be provided by using at least one of a voice output, a visual output, or a tactile output.

As further used herein, the term "scanning" or any grammatical variation thereof refers to a process of capturing at least one image which covers a portion of the space at least partially, i.e. partially or fully, surrounding the person and the device. As generally used, the term "image" refers to a two-dimensional graphical representation of at least one object. Herein, the at least one image may, preferably, comprise at least one scene that exhibits at least one visual target. As generally used, the term "scene" refers to a two-dimensional graphical representation of the environment or a portion thereof as captured in the at least one image. Preferably, the scanning process may comprise recording a plurality of images, each covering a portion, in particular a different portion, of the scene, thus, allowing recognizing the at least one visual target from a single image or, preferably, from at least two individual images, thereby improving a quality of a recognizing process.

As already indicated above, the at least one visual target may, particularly, be selected from a number, a letter, or a symbol, that may be suitable for being perceived by the person. According to the present invention, the at least one visual target may comprise a number, a letter, or a symbol on a surface of at least one object being located in the natural environment, wherein the at least one visual target may, specifically, be
- part of a printed product, such as a newspaper, a book, a packaging, a signage, or a poster;
- handwritten text or symbol, such as handwritten on a piece of paper, a cardboard, or a board;
- displayed on at least one mirror; or
- displayed on at least one digital screen, such as a smartphone, a tablet, a computer monitor, or a television screen.

In a preferred embodiment, the invitation as forwarded to the person may comprise initiating the person to look into a particular direction within the room or the portion thereof at which at least one visual target may be located. As already described above, the invitation can be provided by using at least one of a voice output, a visual output, or a tactile output to the person.

As further used herein, the term "recognizing" or any grammatical variation thereof refers to a process of acquiring at least one first piece of information about the at least one visual target and at least one second piece of information about at least one parameter related to the at least one visual target, especially from the at least one image comprising the scene which exhibits the at least one visual target. As generally used, the term "piece of information" refers to at least one item of data about a particular object or a property of the object. In particular, recognizing the at least one visual target may comprise detecting the at least one visual target in the natural environment of the person and/or segmenting the at least one visual target from the natural environment of the person. As generally used, the term "detecting" or any grammatical variation thereof refers to a process of identifying at least one object, preferably by segmenting the at least one object from a scene, wherein the term "segmenting" or any grammatical variation thereof relates to extracting a particular object from a scene which illustrates an environment comprising a plurality of objects. Herein, the at least one visual target may, initially, be extracted from the scene, thereby providing the at least one first piece of information, whereas the at least one parameter related to the at least one visual target may, subsequently, be determined from the at least one visual target as extracted from the scene, thereby providing the at least one second piece of information.

Herein, at least one particular parameter may directly be determined from the at least one visual target, while at least one other particular parameter may indirectly be determined from the at least one visual target by using at least one additional piece of information, wherein the at least one additional piece of information may, specifically, be a distance between the person and the at least one visual target. Herein, the method for determining the visual performance of the at least one eye of the person may, further, comprise step h) as follows:
h) measuring a distance between the at least one visual target and the at least one eye of the person.

In particular, the visual performance of the at least one eye of the person may be determined by using a measured value for the distance between the at least one visual target and the at least one eye of the person.

Herein, at least one particular parameter, such as an angular size of the at least one visual target, can directly be determined from the at least one visual target, while at least one other particular parameter, such as a lateral extension of the at least one visual target, can indirectly be determined from the at least one visual target by using at least one additional piece of information, such as a distance between the person and the at least one visual target. By way of example, a letter "E" may, initially, be extracted from a book cover as the first piece of information, whereas the angular size, such as an angular width or height, of the letter "E" can, subsequent, directly be derived from the image comprising the extracted letter, while the distance between the person and the book cover may, further, be used for determining the lateral extension of the letter "E". However, various further examples are conceivable.

In a particularly preferred embodiment, recognizing the at least one first piece of information about the at least one visual target may comprise at least one algorithm configured for text recognition, such as an optical character recognition algorithm, also abbreviated to "OCR". As generally used, the term "text recognition" refers to determining at least one of an individual number, an individual letter, or an individual symbol from a line of printed text that may be used as the at least one visual target. Further, at least one algorithm may be configured for handwritten text recognition, in particular for determining at least one piece of handwritten text that may, further, be used as the at least one visual target. Further, at least one algorithm may be configured for text recognition, wherein the text used as the at least one visual target may be displayed on at least one digital screen, such as a smartphone, a tablet, a computer monitor, or a television screen. In addition, at least one algorithm may, further, be configured for pattern recognition. As generally used, the term "pattern recognition" refers to determining at least one pattern that comprises the desired text that may be used as the at least one visual target. Further, recognizing the at least one first piece of information about the at least one visual target may comprise at least one algorithm configured for processing the at least one image comprising the scene which exhibits the at least one visual target. Specifically, at least one of the following exemplary algorithms may be used for executing the respectively denoted tasks:
- an algorithm configured for pattern recognition as disclosed in at least one of US 8 352 400 B2, US 7 880 730 B2, US 7 499 891 B2, EP 3 200 123 A1, or WO 2020/73535 A2;
- an algorithm configured for text recognition based on machine learning as disclosed in at least one of US 7 499 588 B2 or CN 110 399 798 A;
- an algorithm configured for text recognition and typo correction as disclosed in CN 111 435 407 A1;
- an algorithm configured for handwritten text recognition as disclosed in at least one of JP 50 140 022 A2, JP 59 002 191 A2, JP 61 213 982 A2, or JP 61 233 881 A2; or
- an algorithm configured for image processing as disclosed in WO 2019/092672 A2, or known to the person skilled in the art as structure-from-motion (SfM) mapping or simultaneous localization and mapping (SLAM).
However, using at least one further algorithm for any one of the indicated tasks or at least one further task may also be feasible.

In a particular embodiment, recognizing the at least one visual target may, further, comprise classifying the at least one visual target according to the at least one parameter related to the at least one visual target. As used herein, the term "classifying" or any grammatical variation thereof refers to a process, typically denoted as "classification process", of sorting the at least one visual target into at least two classes according to the at least one parameter related to the at least one visual target, wherein at least one numerical value, typically denoted as a "score", is adapted for discriminating between two adjacent classes. By way of example, the type of visual target can be sorted according to a contrast, a size, or to a color, in particular, depending on whether determining the visual performance of the at least one eye of the person may be directed to determining the contrast sensitivity, the a visual acuity or the color vision of the at least one eye of the person. However, using a further type of classification process may also be feasible. As a result, using a particular classification process may facilitate determining the desired type of visual performance of the at least one eye of the person.

In this fashion, it may be possible to provide a series of visual targets to be perceived by the at least one eye of the person by, consecutively, using
- the same visual target providing it by using a series of related modified parameters; or
- a series of different visual targets.
By way of example, the same letter "E" or a series of different letters may be provided in a fashion in which at least one particular parameter that may be related to the at least one visual target can, consecutively, be modified, such as by providing a consecutively decreasing contrast or size, or a consecutively altering color. However, various further examples may also be feasible.

In a particular embodiment, the at least one parameter related to the at least one visual target may be modified by using at least one psychometric procedure. As generally used, the term "psychometric procedure" refers to a process of modifying the at least one parameter by using a preceding reaction of the person to at least one preceding parameter. Herein, the at least one parameter may, especially, be modified by using at least one psychological staircase. As generally used, the term "psychological staircase" refers to altering the at least one parameter in a steadily decreasing or an increasing manner, preferably, by using approximated equidistant linear or logarithmic steps. Herein, the at least one psychological staircase may be selected from a BestPEST or an Up-Down staircase. However, using a further kind of psychological staircase may also feasible.

In a further embodiment, the method for determining the visual performance of the at least one eye of the person may, further, comprise step f) as follows:
f) detecting at least one irregular movement in the face of the person, wherein the at least one irregular movement indicates visual strain of the at least one eye of the person, and classifying the at least one irregular movement using a score related to the visual performance of the at least one eye of the person.

Herein, the classification process may, in particular comprise detecting at least one irregular movement in the face of the person, wherein the at least one irregular movement may indicate visual strain of the at least one eye of the person and, subsequently, classifying the at least one irregular movement, specifically, by using a score related to the visual performance of the at least one eye of the person. For the term "score", reference can be made to the description above. By way of example, a first type of reaction of the person to the visual target which may be obtained by monitoring at least one measurement signal as provided by at last one input unit and, concurrently, a second type of reaction of the person to the visual target can be obtained at the same point in time by observing an alteration in the face of the person, specifically at least one half-closed eye or at least one squinted eye. Herein, both the first type and the second type of the reaction of the person to the visual target can be used for determining the visual performance, specifically, the visual acuity of at least one eye of a person.

According to step b), the visual performance of the at least one eye of the person is determined by using at least one first piece of information about the at least one visual target and at least one second piece of information about at least one parameter related to the at least one visual target at the point in time. In accordance with the present invention, both the at least one first piece of information and the at least one second piece of information are recognized for the same point in time which specifies a particular moment in which the particular reaction of the person indicates that the person is able to perceive a particular visual target having the at least one particular parameter related to the particular visual target. Consequently, all relevant information is available and can, therefore, be used for determining the visual performance of the at least one eye of the person in a manner as known to the person skilled in the art. Preferably, the method for determining the visual performance of the at least one eye of a person may, further, comprise step d) as follows:
d) repeating steps a) to c) by using at least one of:
   - a modified parameter related to the at least one visual target, or
   - a different visual target.
In particular, steps a) to c) may be repeated until all visual targets that can be detected in the natural environment of the person may have been used for the purposes of the present invention, or until a number of visual targets that may have been detected in the natural environment of the person may be sufficient for determining the visual performance of the at least one eye of the person according to the present invention.

In a particularly preferred embodiment, the method for determining the visual performance of the at least one eye of the person may comprise step e) as follows:
e) comparing the at least one first piece of information about the at least one visual target and at least one second piece of information about at least one parameter related to the at least one visual target as determined from the reaction of the person at the point in time with the at least one first piece of information about the at least one visual target and at least one second piece of information about at least one parameter related to the at least one visual target as recognized by scanning the natural environment of the person.

As used herein, the term "comparing" or any grammatical variation thereof refers to a process of determining whether a difference may exist or not between, on one hand, at least one property of the at least one visual target as determined from the reaction of the person at the point in time and, on the other hand, the same property of the at least one visual target as recognized by scanning the natural environment of the person. By way of example, scanning the natural environment of the person may indicate that a particular book cover may comprise the letter "E" as a first letter while, at the same point in time, a microphone as comprised by a mobile communication device may receive a reaction of the person that the particular book cover may comprise the letter "F" as the first letter, in which event a difference exists, from which it can be determined that the visual performance of the eyes of the person are not sufficient to correctly perceive the letter "E" having the related at least one parameter, such as the contrast, the size and/or the color. Based on such a result, an invitation could, further, be provided to the person. wherein the invitation may request the person to approach or to depart from the particular book and, thereafter, to provide a further reaction which may, again, be compared with the result of the scanning of the natural environment of the person, preferably until no difference may exist. However, various further examples are conceivable.

In general, determining the visual performance of the at least one eye of the person according to the present invention can be performed in real time. However, alternatively or in addition, determining the visual performance of the at least one eye of the person can be performed at a later time, in an on-line fashion or an off-line fashion.

In a further preferred embodiment, the method for determining the visual performance of the at least one eye of the person may, further, comprise step g) as follows:
g) generating at least one piece of output information to the person.
As used herein, the term "output information" refers to a piece of data which is transmitted to the person via at least one output channel. For this purpose, at least one of a voice output, a visual output, or a tactile output may be used as the at least one output channel; however, further kinds of output channels may also be feasible. Additionally, the at least one piece of output information may, further, be transmitted to at least one storage unit. As generally used, the term "storage unit" may be a facility configured to receive and maintain the at least one piece of information for further retrieval. Preferably, the at least one storage unit may be at least one external storage unit which may, specifically, be accessible via at least one network, such as an in-house network or the internet.

In a further preferred embodiment, the method for determining the visual performance of the at least one eye of the person may, further, comprise step i) as follows:
i) measuring a luminance level of the at least one visual target.
As generally used, the term "luminance level" refers to an intensity of lighting as received by an object, such as the at least one of a visual target or a luminance sensor configured to measure a luminance level of the visual target. Based on at least one further piece of data related to the luminance level of the at least one visual target, determining the visual performance of the at least one eye of the person may comprise using a measured value for the luminance level of the at least one visual target. In this fashion, the contrast sensitivity can be determined as an absolute contrast sensitivity by, additionally, taking into account the luminance level of the at least one visual target.

In a further preferred embodiment, the method for determining the visual performance of the at least one eye of the person may, further, comprise step k) as follows:
k) initiating a process for producing at least one spectacle lens using the at least one refractive error of the at least one eye of the person.
For further details with respect to the term "refractive error", reference may be made to the description above. As generally used, the term "initiating" or any grammatical variation thereof refers to commencing a process by which it is intended to achieve a particular result. Herein, initiating the process for producing the at least one spectacle lens can, directly, be performed by the person asking for the eyeglasses or by an eye care professional, such as the optician, who forwards the pieces of data comprising the at least one refractive error of the at least one eye of the person together with line of sight information and information about the spectacle frame. For selecting a particular model of spectacle frame for being used in the envisaged eyeglasses, a plurality of spectacle frames can, preferably, be presented to the person on at least one screen, in particular, the at least one screen as comprised by the device according to the present invention.

As an alternative, the person can forward these pieces of data to a further person or body, such as an ophthalmologist or an insurance company, particularly for reviewing the data, and, subsequently, initiating the process for producing the at last one spectacle lens or, as a further alternative, approving the data and enabling the initiating of the process for producing the at last one spectacle lens by the person asking for the eyeglasses or by the eye care professional, such as the optician. However, further alternatives are conceivable.

In a further aspect, the present invention relates to a computer program comprising instructions which, when the program is executed by a computer, cause the computer to carry out any one of the methods according to the present invention. Specifically, the computer program may be stored on a computer-readable data carrier. Thus, specifically, any one of the method steps as indicated above may be performed by using a computer or a computer network, preferably by using a computer program.

In a further aspect, the present invention relates to a computer program product having program code means, in order to perform the methods according to the present invention when the program is executed on a computer or computer network. Specifically, the program code means may be stored on a computer-readable data carrier.

In a further aspect, the present invention relates to a data carrier having a data structure stored thereon, which, after loading into a computer or computer network, such as into a working memory or main memory of the computer or computer network, may execute any one of the methods according to the present invention.

In a further aspect, the present invention relates to a computer program product with program code means stored on a machine-readable carrier, in order to perform the methods according to one or more of the embodiments disclosed herein, when the program is executed on a computer or computer network. As used herein, the term "computer program product" refers to the program as a tradable product. The product may generally exist in an arbitrary format, such as in a paper format, or on a computer-readable data carrier. Specifically, the computer program product may be distributed over a data network, such as the internet.

In a further aspect, the present invention relates to a modulated data signal which comprises instructions readable by a computer system or computer network, for performing any one of the methods according to the present invention.

In a further aspect, the present invention relates to a method for producing at least one spectacle lens for at least one eye of a person. As generally, the producing of the at least one spectacle lens comprises processing at least one lens blank by adjusting at least one optical parameter of the at least one spectacle lens, wherein the processing of the lens blank is based on instructions which are configured to compensate the at least one refractive error of the at least one eye of a person as described in the prior art. According to the present invention, the determining of the visual performance of the at least one eye of a person comprises applying the method for determining the visual performance of the at least one eye of a person according to the present invention.

For further details concerning the method for producing at least one spectacle lens, reference may be made to the method for determining a visual performance of at least one eye of a person as disclosed elsewhere herein.

In a further aspect, the present invention relates to a device for determining a visual performance of at least one eye of a person. Herein, the device comprises
- at least one scanning unit, wherein the at least one scanning unit is configured to scan a natural environment of the person; and
- at least one processing unit, wherein the at least one processing unit is configured to
   ∘ identify a point in time indicating that a person is able to perceive at least one visual target from a reaction of the person;
   ∘ recognize the at least one visual target and the at least one parameter related to the at least one visual target at the point in time by scanning a natural environment of the person; and
   ∘ determine a visual performance of at least one eye of the person by using at least one first piece of information about the at least one visual target and at least one second piece of information about at least one parameter related to the at least one visual target at the point in time.

In a particularly preferred embodiment, the device according to the present invention may be selected from at least one of:
- a virtual reality headset;
- an augmented reality overlay; or
- a mobile communication device, wherein the mobile communication device may, preferably, be selected from at least one of a smartphone, a tablet; or a laptop.
However, a further kind of device may also be conceivable. As further generally used, the term "virtual reality headset" refers to a head-mounted apparatus which is designated for providing virtual reality for the person who wears the virtual reality headset. Herein, the virtual reality headset may, preferably, comprise a stereoscopic head-mounted display which may be capable of providing separate images for each eye; stereo sound; at least one head motion tracking sensor, such as a gyroscope, an accelerometer, or a structured light system; and an eye tracking sensor. For the term "mobile communication device", reference may be made to the definition above. In addition, the mobile communication device may comprise least one sensor, in particular, selected from a depth sensor, or an acceleration and position sensor. However, further kinds of sensors may also be feasible.

As generally used, the term "processing unit" refers to an apparatus which is designated for determining at least one desired value as output item by using a plurality of data as input item. For this purpose, the processing unit may comprise or be comprised by at least one of an integrated circuit, in particular an application-specific integrated circuit (ASIC) or a digital processing unit, specifically at least one of a digital signal processor (DSP), a field programmable gate array (FPGA), a microcontroller, a microcomputer, a computer, or a processing unit, such as comprised by a mobile communication device. Further components may be feasible, in particular at least one of a data acquisition unit, a preprocessing unit, or a storage unit, wherein the at least one data acquisition unit may, preferably, be used for acquiring the plurality of data, while the at least one preprocessing unit may, preferably, be used for preparing the plurality of data to be processed by the processing unit, whereas the at least one storage unit may, preferably, be used for storing at least one of the plurality of data or the desired value. The processing unit may, preferably, be configured to perform at least one computer program, in particular at least one computer program performing or supporting the determining of the at last one desired value, wherein the processing of the data may be performed in a consecutive, a parallel, or a combined fashion. The at least one processing unit is configured to perform the tasks which are indicated above; however, further tasks, irrespective whether they are disclosed herein or not, may also be performed by using the at least one processing unit.

Alternatively or in addition, the at least one processing unit can be configured to have performed at least one of the tasks, irrespective whether they are disclosed herein or not, at least partially, i.e. partially or fully, at at least one remote location, preferably comprising a local server, a remote server, or a cloud server, wherein the remote location may be accessible via a network, in particular an in-house network or via internet. For this purpose, at least one communication unit as comprised by the device, in particular by the mobile communication device, can be used. In this fashion, at least one algorithm configured for image processing, for text recognition, specifically optical character recognition (OCR), for handwritten text recognition, or for pattern recognition which are available at the remote location may be used. However, further tasks can also be performed in this manner.

As further generally used, the term "scanning unit" refers to an apparatus which is designated for capturing at least one image that covers a portion of the natural environment of the person and the device, wherein the at least one image may, in particular, comprise at least one scene that may exhibit the at least one visual target. Preferably, the scanning unit may be selected from at least one of a camera, a depth sensor, or an acceleration and position sensor. However, a further kind of scanning unit may also be conceivable. Preferably, the at least one camera may comprise a high-resolution camera sensor in order to provide a high spatial and/or temporal resolution of virtual targets as recognized in the natural environment. However, using a further type of camera may also be feasible. In particular, the at least one camera may be selected from at least one of a front camera or a back camera as comprised by the mobile communication device, wherein using the at least one back camera may be preferred.

Preferably, the at least one depth sensor may be selected from at least one of a LIDAR sensor, a time-of-flight sensor, an ultrasonic sensor, a triangulation sensor, a stereo sensor, a structured light sensor, a FIP sensor; or a BPA sensor, such as known to the person skilled in the art. For the FIP sensor, reference can, for example, be made to WO 2012/110924 A1, WO 2014/ 097181 A1, or WO 2016/ 120392 A1. For the BPA sensor, reference can, for example, be made to WO 2018/091640 A2. However, a further kind of depth sensor may also be feasible.

In a further embodiment, the device may, additionally, comprise at least one luminance sensor, wherein the luminance sensor may be configured to measure a luminance level of the at least one visual target. For this purpose, the at least one scanning unit, preferably, the at least one camera as comprised by the device, in particular the mobile communication device, can be used as the luminance sensor, wherein the at least one processing unit may, further, be configured to
∘ determine the visual performance of the at least one eye of the person by using a measured value for the luminance level of the at least one visual target.

In a particularly preferred embodiment, the device may, further, comprise at least one input unit being configured to record the reaction of the person to the at least one visual target. As generally used, the term "input unit" refers to an apparatus which is configured to monitor an occurrence of an event by providing or interrupting at least one measurement signal at a point of time at which the event occurs. With particular regard to the present invention, the at least one reaction of the person can be recorded by using at least one input unit, wherein the at least one input unit may be configured to record a particular kind reaction of the person to the at least one visual target.

In a preferred embodiment, the at least one input unit can be a keyboard, which may comprise at least one key or button to be pressed by the person in order to express the reaction. Herein, the at least one keyboard may be at least one of a real keyboard or, preferably, a virtual keyboard, such as comprised by a touchscreen of a smartphone. Alternatively or in addition, the at least one input unit may comprise at least one microphone, preferably, the at least one microphone that may be comprised by a mobile communication device, which may be configured to generate at least one measurement signal which may indicate the desired reaction of the person.

As a further alternative or in addition, the at least one input unit may be configured to determine the desired reaction of the person by using gesture recognition. For this purpose, the at least one scanning unit, preferably, the at least one camera as comprised by the device, in particular the mobile communication device, can, in particular, be used in combination with at least one algorithm configured to identify the reaction of the person from a non-verbal expression of the person, specifically from an alteration in the face of the person that can be interpreted as the desired reaction of the person.

As a still further alternative or in addition, the at least one input unit may be configured to determine the desired reaction of the person by observing at least one type of eye movements in the at least one eye of the person that may be elicited by the at least one visual target. For this purpose, the at least one scanning unit, preferably, the at least one camera as comprised by the device, in particular the mobile communication device, can, in particular, be used as an eye tracker in combination with at least one algorithm configured to track the eye movements in the at least one eye of the person. However, using a separate eye tracker, which may, preferably, be mounted at a side of the at least one eye of the person, may also be feasible.

In a preferred embodiment, the device may, further, comprise at least one screen, such as the at least one screen as comprised by a mobile communication device. Herein, the at least one screen may, preferably, be designated for displaying to the at least one eye of the person at least one of:
- the at least one visual target according to the at least one parameter related to the at least one visual target;
- at least one numeric value or alphanumerical value related to
   ∘ the visual performance of at least one eye of the person;
   ∘ at least one refractive error of the at least one eye of the person;
- at least one suggestion for initiating a process for producing at least one spectacle lens based on the at least one refractive error of the at least one eye of the person.

However, the at least one screen may also be designated for displaying to the at least one eye of the person at least one further kind of item. By way of example, a plurality of visual targets, each having at least one different parameter related to each visual target, may be displayed, in a simultaneous or a consecutive fashion, on at least one screen.

In a particular embodiment, the device may, further, comprise at least one mirror, wherein the mirror may be designated for displaying a mirrored version of the at least one visual target to the at least one eye of the person.

In a further embodiment, the device may, additionally, comprise at least one distance measuring unit, wherein the distance measuring unit may be designated for determining a distance between the at least one visual target and the at least one eye of the person. For this purpose, the at least one scanning unit, preferably, the at least one camera as comprised by the device, in particular the mobile communication device, can, in particular, be used as the distance measuring unit, wherein the at least one processing unit my, further, comprise at least one algorithm being configured to determine the distance between the at least one visual target and the at least one eye of the person.

For further details concerning the device for determining a visual performance of at least one eye of a person, reference may be made to the description of the method for determining a visual performance of at least one eye of a person as provided elsewhere herein.

With respect to the prior art, the method, the computer program and the device for determining the visual performance of the at least one eye of the person according to the present invention exhibit various advantages. Accordingly, no additional external devices, such an acuity board and/or an external screen are needed for performing the present invention. As a result, vision testing can be performed without a need of experts and as a home screening procedure. Further, it is not necessary to evaluate the reaction of the person and/or to determine the visual performance in real time; rather the present invention allows an evaluation of the reaction of the person and/or of the visual performance at a later time, in particular also in an off-line fashion.

As used herein, the terms "have", "comprise" or "include" or any grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following Embodiments are particularly preferred within the scope of the present invention:
Embodiment 1. A computer-implemented method for determining a visual performance of at least one eye of a person, the method comprising the following steps:
   a) identifying a point in time indicating that a person is able to perceive at least one visual target from a reaction of the person;
   b) determining a visual performance of at least one eye of the person by using at least one first piece of information about the at least one visual target and at least one second piece of information about at least one parameter related to the at least one visual target at the point in time; and
   c) recognizing the at least one visual target and the at least one parameter related to the at least one visual target at the point in time by scanning a natural environment of the person.
Embodiment 2. The method according to the preceding Embodiment, wherein the method is performed in an order:
   - commencing with step a);
   - continuing with step c); and
   - finishing with step b).
Embodiment 3. The method according to any one of the preceding Embodiments, wherein the visual performance is selected from at least one of:
   - a contrast sensitivity;
   - a visual acuity;
   - a color vision; or
   - a visual field.
Embodiment 4. The method according to the preceding Embodiment, wherein the visual performance is selected from at least one of a static visual performance or a dynamic visual performance.
Embodiment 5. The method according to any one of the preceding Embodiments, wherein the at least one visual target is selected from at least one of:
   - a number;
   - a letter; or
   - a symbol.
Embodiment 6. The method according to any one of the preceding Embodiments, wherein the at least one parameter related to the at least one visual target is selected from at least one of:
   - a size;
   - an angular size;
   - a contrast;
   - a contrast gradient;
   - a color;
   - a color gradient;
   - a spatial position;
   - a velocity of movement; or
   - a distance to the person of the at least one visual target.
Embodiment 7. The method according to any one of the preceding Embodiments, wherein recognizing the at least one visual target comprises at least one of:
   - detecting at least one object in the natural environment of the person; or
   - segmenting at least one object from the natural environment of the person.
Embodiment 8. The method according to any one of the preceding Embodiments, wherein recognizing the at least one visual target and the at least one parameter related to the at least one visual target comprises:
   - identifying the at least one visual target; and
   - determining the at least one parameter related to the at least one visual target from at least one scan of the at least one object.
Embodiment 9. The method according to any one of the preceding Embodiments, wherein recognizing the at least one visual target comprises classifying the at least one visual target according to the at least one parameter related to the at least one visual target.
Embodiment 10. The method according to any one of the preceding Embodiments, wherein identifying the point in time indicating that the person is able to perceive the at least one visual target comprises recording the reaction of the person to the at least one visual target.
Embodiment 11. The method according to any one of the preceding Embodiments, wherein the reaction of the person to the at least one visual target is selected from at least one of:
   - a touch response;
   - a voice entry;
   - gesture recognition; or
   - an eliciting of at least one type of eye movements in the at least one eye of the person.
Embodiment 12. The method according to the preceding Embodiment, wherein the at least one type of eye movements is selected from at least one of:
   - fixational eye movements;
   - pursuit eye movements; or
   - an optokinetic nystagmus.
Embodiment 13. The method according to the preceding Embodiment, wherein tracking the at least one optokinetic nystagmus in the at least one eye of the person comprises a saccade detection and a slow phase velocity thresholding.
Embodiment 14. The method according to any one of the preceding Embodiments, further comprising a step of:
   d) repeating steps a) to c) by using at least one of:
      - a modified parameter related to the at least one visual target, or
      - a different visual target.
Embodiment 15. The method according to any one of the preceding Embodiments, wherein at least one of:
   - the at least one parameter related to the at least one visual target, or
   - the at least one modified parameter related to the at least one visual target is selected by using at least one psychometric procedure.
Embodiment 16. The method according to any one of the preceding Embodiments, wherein determining the visual performance of the at least one eye of the person comprises a step of:
   e) comparing the at least one visual target and the at least one parameter related to the at least one visual target as recognized by scanning the natural environment of the person with at least one further piece of information about the at least one visual target at the point in time as determined from the reaction of the person.
Embodiment 17. The method according to any one of the preceding Embodiments, wherein determining of the visual performance of the at least one eye of the person comprises a step of:
   f) detecting at least one irregular movement in the face of the person, wherein the at least one irregular movement indicates visual strain of the at least one eye of the person, and classifying the at least one irregular movement using a score related to the visual performance of the at least one eye of the person.
Embodiment 18. The method according to any one of the preceding Embodiments, further comprising a step of:
   g) generating at least one piece of output information to the person.
Embodiment 19. The method according to the preceding Embodiment, wherein the at least one piece of output information is transmitted to the person by using at least one of:
   - a voice output;
   - a visual output; or
   - a tactile output.
Embodiment 20. The method according to the preceding Embodiment, wherein the at least one piece of output information is further transmitted to at least one storage unit.
Embodiment 21. The method according to the preceding Embodiment, wherein the at least one storage unit is accessible via at least one network.
Embodiment 22. The method according to any one of the preceding Embodiments, further comprising a step of:
   h) measuring a distance between the at least one visual target and the at least one eye of the person.
Embodiment 23. The method according to the preceding Embodiment, wherein the visual performance of the at least one eye of the person is determined by using a measured value for the distance between the at least one visual target and the at least one eye of the person.
Embodiment 24. The method according to any one of the preceding Embodiments, further comprising a step of:
   i) measuring a luminance level of the at least one visual target.
Embodiment 25. The method according to the preceding Embodiment, wherein the visual performance of the at least one eye of the person is determined by using a measured value for the luminance level of the at least one visual target.
Embodiment 26. The method according to any one of the preceding Embodiments, further comprising a step of:
   j) determining at least one refractive error of the at least one eye of the person by using the visual performance of the at least one eye of the person.
Embodiment 27. The method according to any one of the preceding Embodiments, further comprising a step of:
   k) initiating a process for producing at least one spectacle lens using the at least one refractive error of the at least one eye of the person.
Embodiment 28. A computer program comprising instructions which, when the program is executed by a computer, cause the computer to carry out the method for determining a visual performance of at least one eye of a person according to any one of the preceding method steps.
Embodiment 29. A method for producing at least one spectacle lens, wherein producing the at least one spectacle lens comprises processing at least one lens blank by using at least one centration parameter, wherein the at least one centration parameter is determined by using a method for determining a visual performance of at least one eye of a person according to any one of the preceding method steps.
Embodiment 30. The method according to the preceding Embodiment, wherein the at least one centration parameter is determined by using at least one refractive error of the at least one eye of the person as determined from the visual performance of the at least one eye of the person.
Embodiment 31. A device for determining a visual performance of at least one eye of a person, the device comprising:
   - at least one scanning unit, wherein the at least one scanning unit is configured to scan a natural environment of the person; and
   - at least one processing unit, wherein the at least one processing unit is configured to:
      ∘ identify a point in time indicating that a person is able to perceive at least one visual target from a reaction of the person;
      ∘ recognize the at least one visual target and the at least one parameter related to the at least one visual target at the point in time by scanning a natural environment of the person; and
      ∘ determine a visual performance of at least one eye of the person by using at least one first piece of information about the at least one visual target and at least one second piece of information about at least one parameter related to the at least one visual target at the point in time.
Embodiment 32. The device according to the preceding Embodiment, wherein the at least one scanning unit is selected from at least one of:
   - a camera;
   - a depth sensor; or
   - an acceleration and position sensor.
Embodiment 33. The device according to the preceding Embodiment, wherein the at least one depth sensor is selected from at least one of:
   - a LIDAR sensor;
   - a time-of-flight sensor;
   - an ultrasonic sensor;
   - a triangulation sensor;
   - a stereo sensor;
   - a structured light sensor;
   - a FIP sensor; or
   - a BPA sensor.
Embodiment 34. The device according to any one of the preceding device Embodiments, further comprising:
   - at least one input unit, wherein the at least one input unit is configured to record the reaction of the person to the at least one visual target.
Embodiment 35. The device according to any one of the preceding device Embodiments, further comprising:
   - at least one screen.
Embodiment 36. The device according to the preceding Embodiment, wherein the at least one screen is designated for displaying to the at least one eye of the person at least one of:
   - the at least one visual target according to the at least one parameter related to the at least one visual target;
   - at least one numeric value or alphanumerical value related to
      ∘ the visual performance of at least one eye of the person;
      ∘ at least one refractive error of the at least one eye of the person;
   - at least one suggestion for initiating a process for producing at least one spectacle lens based on the at least one refractive error of the at least one eye of the person.
Embodiment 37. The device according to any one of the preceding device Embodiments, further comprising:
   - a mirror, wherein the mirror is designated for displaying a mirrored version of the at least one visual target to the at least one eye of the person.
Embodiment 38. The device according to any one of the preceding device Embodiments, further comprising:
   - a distance measuring unit, wherein the distance measuring unit is designated for determining a distance between the at least one visual target and the at least one eye of the person.
Embodiment 39. The device according to any one of the preceding device Embodiments, further comprising:
   - a luminance sensor, wherein the a luminance sensor is configured to measure a luminance level of the at least one visual target,
Embodiment 40. The device according to the preceding Embodiment, wherein the at least one processing unit is further configured to:
   ∘ determine the visual performance of the at least one eye of the person by using a measured value for the luminance level of the at least one visual target.
Embodiment 41. The device according to any one of the preceding device Embodiments, wherein the device is selected from at least one of:
   - a virtual reality headset;
   - an augmented reality overlay; or
   - or a mobile communication device.
Embodiment 42. The device according to the preceding Embodiment, wherein the mobile communication device is selected from at least one of:
   - a smartphone;
   - a tablet; or
   - a laptop.
Embodiment 43. The device according to any one of the preceding device Embodiments, further comprising:
   - at least one communication unit, wherein the at least one communication unit is configured to performing a data exchange with at least one remote location.
Embodiment 44. The device according to the preceding Embodiment, wherein at least one of:
   - a local server;
   - a remote server; or
   - a cloud server
      is located at the at least one remote location.

### Short description of the Figures

Further optional features and embodiments of the present invention are disclosed in more detail in the subsequent description of preferred embodiments, preferably in conjunction with the dependent claims. Therein, the respective optional features may be implemented in an isolated fashion as well as in any arbitrary feasible combination, as the skilled person will realize. It is emphasized that the scope of the invention is not restricted by the preferred embodiments. In the Figures:
- Figure 1: illustrates a preferred exemplary embodiment of a device for determining a visual performance of at least one eye of a person located in a natural environment according to the present invention; and
- Figure 2: illustrates a preferred exemplary embodiment of a method for determining a visual performance of at least one eye of a person according to the present invention.

### Detailed description of the embodiments

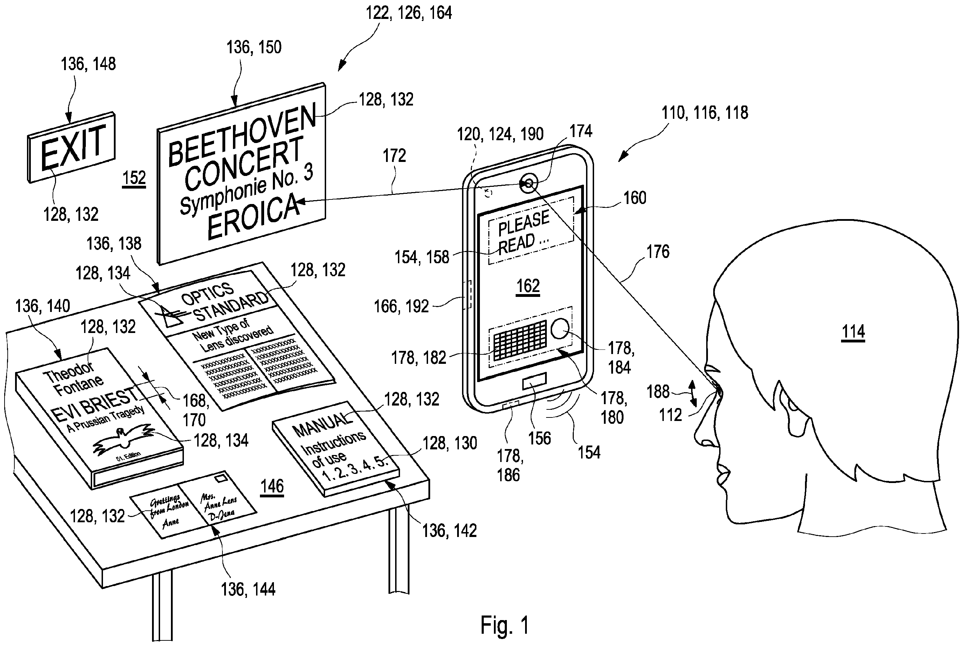

Figure 1 shows a preferred exemplary embodiment of a device 110 for determining a visual performance of one or both eyes 112 of a person 114. In the illustration according to Figure 1 and in the following description, the device 110 is implemented - without limiting the generality of the invention - as a mobile communication device 116 in form of a smartphone 118. However, implementing the device 110 in form of a virtual reality headset, an augmented reality overlay or a different type of mobile communication device 116, in particular a tablet or a laptop may also feasible.

As already defined above, the visual performance refers to a property of the one or both eyes 112 of the person 114, which can be determined by investigating one or both eyes 112 of the person 114 using an adapted measurement procedure. For the purposes of the present invention, the visual performance can be selected from one or more properties of the one or both eyes 112 of the person 114, in particular from a contrast sensitivity, a visual acuity, a color vision, or a visual field of the one or both eyes 112 of the person 114. For the respective terms, reference can be made to the definitions as provided above. Furthermore, each of the mentioned properties can, alternatively or in addition, be determined in a static fashion and/or a dynamic fashion.

As further illustrated in Figure 1, the smartphone 118 comprises a scanning unit 120 being configured to scan a natural environment 122 which surrounds the person 114 and the smartphone 118. In this exemplary embodiment, a back camera 124 of the smartphone 118 is used here as the scanning unit 120 for scanning the natural environment 122. As schematically depicted in Figure 1, the natural environment 122 surrounding the person 114 and the smartphone 118 is a portion of a room 126. Herein, the room 126 may be a personal room in an accommodation at which the person 114 may reside, a room in a shop of optician, or a room in a practice of an ophthalmologist; however, further rooms may also be feasible. To facilitate maintaining a constant luminance level, the natural environment 122 may be an indoor environment, however, using an outdoor environment may also be feasible.

As further illustrated in Figure 1, the natural environment 122 surrounding the person 114 and the smartphone 118 comprises one or more visual targets 128. As generally used, the visual targets 128 may be selected from a number 130, a letter 132, or a symbol 134, which may be suitable for being perceived by the person 114. Herein, the number 130, the letter 132, or the symbol 134 may correspond to or be similar to visual targets that have, typically, been used in known standard vision tests familiar to the person skilled in the art over a long period of time, however, further kinds of visual targets may also be feasible.

According to the present invention, the visual targets 128 may be placed on a surface of one or more objects 136 which are located in the natural environment 122. As schematically depicted in Figure 1, the objects 136 comprise a part of a newspaper 138, of a book 140, of a packaging 142, or of a handwritten postcard 144 lying on a table 146 being located in the portion of the room 126, or of a signage 148 or of a poster 150 hanging on a wall 152 bordering the portion of the room 126. However, various other kinds of visual targets 128 may also be feasible, in particular, one or more visual targets 128 that may be digitally displayed on a digital screen, such as a smartphone, a tablet, a computer monitor, or a television screen. In a particular embodiment, the portion of the room 126 may comprise one or more mirrors (not depicted here), wherein the mirror may be designated for displaying a mirrored version of one or more visual targets 128 to the one or both eyes 112 of the person 114.

According to the present invention, the natural environment 122 can, preferably, be used "as is", which indicates that the portion of the room 126 already comprises sufficient visual targets 128 such that no prior preparation of the portion of the room 126 may be required. However, one or more further visual targets 128 can, additionally, be placed in the portion of the room 126, in particular if the portion of the room 126 may not comprise a sufficient number of suitable visual targets 128. In this event, the smartphone 118 can provide an invitation 154 to initiate the person 114 or any other person, in particular an optician, an ophthalmologist or an assistant, to place one or more further visual targets 128 within the room or the portion thereof in a desired fashion. For this purpose, the invitation 154 can be provided to the person 114 by using a loudspeaker 156 comprised by the smartphone 118. Alternatively or in addition, the invitation can be provided to the person 114 by using a text message 158 that can be provided to the person 114 on an output area 160 on a touch-sensitive screen 162 as further comprised by the smartphone 118. Further alternatives, such as a tactile output, may also be feasible.

As already indicated above, the back camera 124 of the smartphone 118 is used in this exemplary embodiment as the scanning unit 120 for scanning the natural environment 122. As a result of a scanning process, one or more images are captured that cover the portion of the room 126 in which the visual targets 128 are located. In this fashion, the one or more images comprise one or more scenes 164 exhibit one or more of the visual targets 128. Preferably, the scanning process may comprise recording a plurality of images, each, preferably, covering a different portion of the scene 164.

Further, the smartphone 118 comprises a processing unit 166 that is configured to recognize the one or more visual targets 128 by scanning the natural environment 122 of the person 114 and to determine one or more parameters 168 related to the one or more visual targets 128 from the one or more visual targets 128 as scanned in this fashion. In particular, recognizing the one or more visual targets 128 may comprise detecting the one or more visual targets 128 in the natural environment 122 of the person 114 by identifying the one or more visual targets 128 within the scene 164 and/or segmenting the one or more visual targets 128 from the natural environment 122 of the person 114 by extracting a particular visual target 128 from the scene 164. For a purpose of recognizing the one or more visual targets 128 within the scene 164, one or more algorithms configured for image processing, for text recognition, specifically optical character recognition (OCR), for handwritten text recognition, or for pattern recognition may be used. For further details, reference can be made to the description above.

It is emphasized here, that the processing unit 166 can, alternatively or in addition, be configured to have performed one or more of the tasks, irrespective whether they are disclosed herein or not, at least partially, i.e. partially or fully, at at least one remote location (not depicted here). Herein, a local server, a remote server, or a cloud server may be used, wherein the remote location may be accessible via one or more networks, in particular an in-house network or via internet. For this purpose, the smartphone 118 can comprise one or more communication units (not depicted here) in order to communicate with the local server, the remote server and/or the cloud server at the remote location. Herein, the one or more algorithms configured for image processing, for text recognition, specifically optical character recognition (OCR), for handwritten text recognition, or for pattern recognition which are available at the remote location may be used. However, further tasks can also be performed in this manner.

As also described above in more detail, each parameter 168 refers to a property of one or more visual targets 128 that is capable to be expressed by one or more numerical or alphanumerical values. As schematically depicted in Figure 1, the one or more parameters 168 may be a size 170, an angular size, a contrast, a contrast gradient, a color and/or a color gradient of the visual targets 128. Further, the one or more parameters 168 may be a spatial position and/or a velocity of a movement of the visual targets 128. Still further, the one or more parameters 168 may be a distance of the one or more visual targets 128 to the person 114. For this purpose, the back camera 124 of the smartphone may be configured to determine a distance 172 to the one or more visual targets 128, while a front camera 174 may be configured to determine a distance 176 to the person 114, wherein the processing unit 166 may, further, be configured to determine the distance of the one or more visual targets 128 to the person 114, such as by using an trigonometric algorithm. Alternatively or in addition, the distances 172, 176 may be determined by using one or more depth sensors (not depicted here) that may, further, be comprised by the smartphone 118. For further details with regard to the depth sensor , reference may be made to the description above. For this purpose, the smartphone 118 may, further, comprise one or more acceleration and position sensors (not depicted here).

It is emphasized here that determining the visual performance of the one or both eyes 112 of the person 114 according to the present invention uses one or more visual targets 128 which are already available in the natural environment 122 surrounding the person 114, wherein the one or more visual targets 128 as well as the one or more parameters 168 related to the one or more visual targets 128 are recognized by scanning the natural environment 122 of the person 114. This kind of procedure is in particular contrast to known processes for determining the visual performance of the one or both eyes 112 of the person 114, in which the one or more visual targets 128 are generated on a screen, such as on the touch-sensitive screen 162 of the smartphone 118, by electronically selecting the one or more visual targets 128 and by electronically determining the one or more parameters 168 related to the one or more visual targets 128 in a fashion to be presented to the person 114.

Further, the processing unit 166 is configured to identify a point in time which indicates that the person 114 is able to perceive one or more particular visual targets 128 from one or more reactions of the person 114. By identifying the point in time one or more numeric values, preferably exactly one numerical value, are generated which specify a particular moment in which the one or more reactions of the person 114 indicate that the person 114 is able to perceive the one or more particular visual targets 128. To support the person 114 to perceive the one or more particular visual targets 128, the invitation 154 that can be forwarded to the person 114, preferably by using the loudspeaker 156 and/or the text message 158, may comprise initiating the person to look into a particular direction within the portion of the room 126 at which the one or more particular visual targets 128 may be located.

The one or more reactions of the person 114 indicate one or more responses of the person 114 as provided by the person 114 upon perceiving or not perceiving the one or more visual targets 128. Herein, the one or more reactions of the person 114 can be recorded by any process which is suitable for determining the one or more reactions of the person 114, either by observing a behavior of the person 114 and/or by monitoring one or more measurement signals as provided by one or more input units 178 configured to generate the one or more measurement signals upon occurrence of the one or more reactions of the person 114.

Preferably, the one or more reactions of the person 114 to the one or more visual targets 128 can be received in form of a touch response by generating the one or more measurement signals by the person 114 touching the touch-sensitive screen 162 of smartphone 118. For this purpose, the touch-sensitive screen 162 may, further, comprise an input area 180 having a virtual keypad 182 and a button 184. Alternatively or in addition, the one or more reactions of the person 114 to the one or more visual targets 128 can be received in form of a voice entry by generating the one or more measurement signals by a microphone 186 as further comprised by the smartphone 118. However, generating the one or measurement signals in a different fashion may also be feasible.

Alternatively or in addition, the one or more reactions of the person 114 to the one or more visual targets 128 can be recorded by observing the behavior of the person 114, in particular by using gesture recognition, specifically by identifying the one or more reactions of the person 114 from an alteration in the face of the person 114 provoked by the one or more visual targets 128 as observed by using the front camera 174 of the smartphone 118. As described above in more detail, an irregular movement in the face of the person 114 indicating a visual strain of the one or both eyes 112 of the person 114 could also be used for this purpose.

Alternatively or in addition, the one or more reactions of the person 114 to the one or more visual targets 128 can be recorded by observing eye movements 188 in the one or both eyes 112 of the person 114 which may be elicited by the one or more visual targets 128. Herein, the eye movements 118 that may be elicited by the one or more visual targets 128 can be one or more of fixational eye movements, pursuit eye movements, or an optokinetic nystagmus. Herein, tracking the optokinetic nystagmus in the one or both eyes 112 of the person 114 may comprise saccade detection and slow phase velocity thresholding. For a purpose of observing the eye movements 188 in the one or both eyes 112 of the person 114, the smartphone 118 may use the front camera 174 of the smartphone 118 as an eye tracker. However, using a separate eye tracker (not depicted here), preferably mounted at a side of the one or both eyes 112 of the person 114, may also be feasible.

In order to support the person 114 and/or to prompt the person 114 to provide the expected one or more reactions in a desired fashion, the invitation 154 to the person 114 may be configured to forward appropriate commands to the person 114. For this purpose, a specific command may be provided to the person 114 to identify a particular visual target 128, such as to read one or more particular letters 132, or to report the content of a particular number 130 or symbol 134 by using the microphone 186 of the smartphone 118. Further, the one or more reactions of the person 114 can be recorded simultaneously for both eyes 112 and/or consecutively for each eye 112. For recording the one or more reactions of the person 114 in a consecutive fashion, the invitation 154 may comprise initiating the person 114 to cover one of the eyes 112 of the person 114.

Further, the processing unit 166 is configured to determine the visual performance of the one or both eyes 112 of the person 114 by using, firstly, one or more first pieces of information about the one or more visual targets 128 and, secondly, one or more second pieces of information about the one or more parameters 168 related to the one or more visual targets 128 as determined at the point in time. In accordance with the present invention, both the one or more first pieces of information and the one or more second pieces of information are recognized for the same point in time which specifies the particular moment in which the particular one or more reactions of the person 114 indicate that the person is able to perceive the one or more particular visual targets 128 which exhibit the one or more particular parameters 168 related to the one or more particular visual targets 128. Consequently, all relevant information is available and can, therefore, be used for determining the visual performance of the one or both eyes 112 of the person 114 in a manner as known to the person skilled in the art.

Further, the processing unit 166 may, preferably, be configured to determine the visual performance of the one or both eyes 112 of the person 114 by using a measured value for a luminance level of the one or more visual targets 128. For this purpose, the smartphone 118 may, further, comprise a luminance sensor 190, which may be configured to measure the luminance level of the one or more visual targets 128. As depicted in Figure 1, the back camera 124 of the smartphone 118 can be used as the luminance sensor 190. In this fashion, the values related to the contrast sensitivity can be determined as an absolute contrast sensitivity value by, additionally, taking into account the luminance level of the one or more visual targets 128 as measured by the luminance sensor 190.

Further, the smartphone 118 can, preferably, be configured to transmit one or more pieces of output information to the person 114 via at least one output channel. For this purpose, the loudspeaker 156 as comprised by the smartphone 118 or, alternatively or in addition, the output area 160 on a touch-sensitive screen 162 as further comprised by the smartphone 118 can be used. Additionally, the one or more pieces of data of output information may, further, be transmitted to one or more storage units 192 as, further, comprised by the smartphone 118; however using one or more external storage units which may, specifically, be comprised by the local server, the remote server and/or the cloud server at the remote location and accessible via accessible via one or more networks, in particular an in-house network or via internet, by using the one or more communication units, may also be feasible.

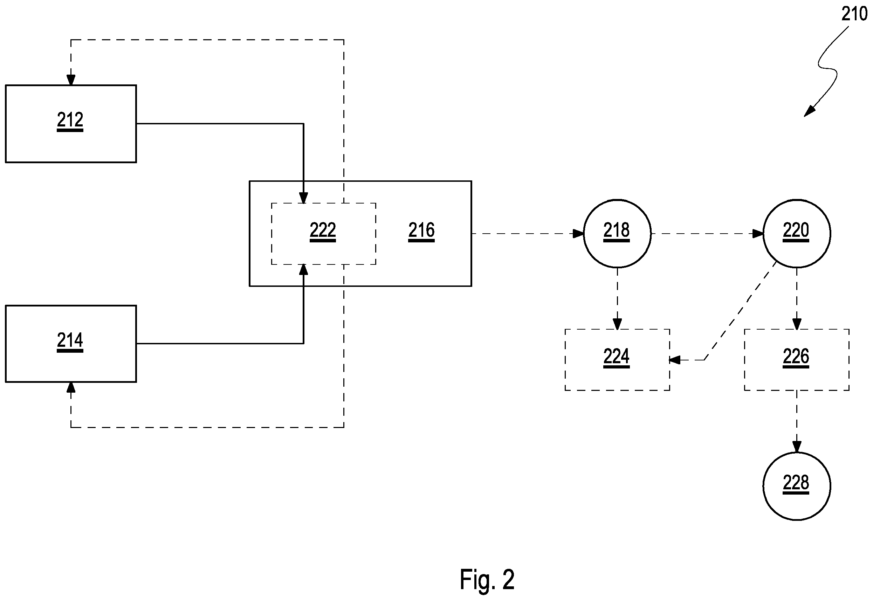

Figure 2 schematically illustrates a preferred embodiment of a computer-implemented method 210 for determining the visual performance of the one or both eyes 112 of the person 114 according to the present invention. As indicated above, the visual performance refers to a property of the one or both eyes 112 of the person 114 which can be determined by investigating the one or both eyes 112 of the person 114 by using an adapted measurement procedure. As described above in more detail, the visual performance can, in particular, be selected from one or more of the contrast sensitivity, the visual acuity, the color vision, or the visual field of the one or both eyes 112 of the person 114. For the respective terms, reference can be made to the definitions as provided above. Furthermore, each of the mentioned properties can, alternatively or in addition, be determined a static fashion and/or a dynamic fashion.

In an identifying step 212 according to step a), the point in time is identified which indicates that the person 114 is able to perceive the one or more visual targets 128 from the one or more reactions of the person 114. As described above in more detail, the one or more reactions of the person 114 can be recorded by any process which is suitable for determining the one or more reactions of the person 114, either by observing the behavior of the person 114 and/or by monitoring the one or more measurement signals as provided by the one or more input units 178 configured to generate the one or more measurement signals upon occurrence of the one or more reactions of the person 114.

In a recognizing step 214 according to step c), the one or more visual targets 128 and the one or more parameters 168 related to the one or more visual targets 128 are recognized for the same point in time by scanning the natural environment 122 of the person 114. For this purpose, the smartphone 118 comprises a scanning unit 120, in particular the back camera 124 of the smartphone 118 and/or the one or more depth sensors, which is configured to scan the natural environment 122. As described above in more detail, the natural environment 122 surrounding the person 114 and the smartphone 118 comprises the one or more visual targets 128.

In a determining step 216 according to step b), the visual performance 218 of the one or both eyes 112 of the person 114 is determined by using the one or more first pieces of information about the one or more visual targets 128 and the one or more second pieces of information about the one or more parameters 168 related to the one or more visual targets 128 as determined at the point in time.

In a particular embodiment, the visual performance 218 of the one or both eyes 112 of the person 114 as determined by using the computer-implemented method 210 as disclosed herein can, preferably, be used for determining one or more further properties of the one or both eyes 112 of the person 114. Specifically, one or more refractive errors 220 of the one or both eyes 112 of the person 114 can be determined from the contrast sensitivity as determined by using the computer-implemented method 210. For further details in this respect, reference can be made to the description above.

For determining the visual performance of the one or both eyes 112 of the person 114, any or all of the identifying step 212, the recognizing step 214 and/or the determining step 216 can be repeated by using one or more modified parameters related to the one or more visual targets 128, or one or more different visual targets 128. Herein, the one or more modified parameters may be obtained by using one or more psychometric procedures, wherein one or more psychological staircases as described above in more detail may be used. However, using a further kind of psychometric procedure may also feasible. In particular, any or all of the identifying step 212, the recognizing step 214 and/or the determining step 216 may be repeated until all visual targets 128 in the natural environment 122 of the person 114 have been detected, or until a number of visual targets 128 that may have been detected in the natural environment 122 of the person 114 may be sufficient for determining the visual performance of the one or both eyes 112 of the person 114. However, further alternatives may also be conceivable.

For determining the visual performance of the one or both eyes 112 of the person 114, the one or more first pieces of information and the one or more second pieces of information as determined from the reaction of the person 114 at the point in time may, preferably, be compared in an optional comparing step 222 with one or more first pieces of information and one or more second pieces of information as recognized by scanning the natural environment 122 of the person 114. In this fashion, it can, firstly, be determined whether the reaction as provided by the person 114 may be correct or not. This result can, subsequently, be used in determining the visual performance of the one or both eyes 112 of the person 114, specifically by taking into account the one or more parameters 168 related to the one or more visual targets 128 as use for this comparison.

By way of example, scanning the natural environment 122 of the person 114 may indicate that the cover of the book 140 comprises, inter alia, the text "EFFI". At the same point in time, the microphone 186 of the smartphone 118 may, however, receive a reaction of the person 114 which indicates that the same text on the cover of the book 140 shall be "FILE". According to this reaction, a difference between the text as determined from scanning the natural environment 122 of the person 114 and the text as determined from the reaction of the person 114 exists, from which it can be determined that the visual performance of the one or both eyes 112 of the person 114 are not sufficient to correctly perceive this text, whereby the one or more parameters 168, such as the contrast, the size and/or the color, which are related to the text are taken into account. However, various further examples are conceivable.

Further, the method for determining the visual performance of the one or both eyes 112 of the person 114 may, further, comprise a generating step 224 of generating at least one piece of output information to the person 114. As described above in more detail, the at least one output channel, specifically, the loudspeaker 156 and/or the output area 160 of the touch-sensitive screen 162 of the smartphone 118 can be used for this purpose.

Further, the method for determining the visual performance of the one or both eyes 112 of the person 114 may, further, comprise an initiating step 226 of initiating a process for producing one or more spectacle lenses 228 by using the one or more refractive errors 220 of the one or both eyes 112 of the person 114 as determined from the contrast sensitivity as determined by using the computer-implemented method 210. Herein a plurality of spectacle frames can, preferably, be presented to the person 114 on the screen 162 in a manner that the person 114 may select a particular model of spectacle frame for being used in the envisaged eyeglasses. As described above in more detail, initiating the process for producing the at one or more spectacle lenses 228 can, directly, be performed by the person 114 or by an eye care professional, such as the optician, by a further person or body, such as an ophthalmologist or an insurance company. However, further alternatives are conceivable.

### List of Reference Signs

- 110: device for determining a visual performance of at least one eye of a person
- 112: eyes
- 114: person
- 116: mobile communication device
- 118: smartphone
- 120: scanning unit
- 122: natural environment
- 124: back camera
- 126: (portion of) room
- 128: visual target
- 130: number
- 132: letter
- 134: symbol
- 136: object
- 138: newspaper
- 140: book
- 142: packaging
- 144: postcard
- 146: table
- 148: signage
- 150: poster
- 152: wall
- 154: invitation
- 156: loudspeaker
- 158: text message
- 160: output area
- 162: (touch-sensitive) screen
- 164: scene
- 166: processing unit
- 168: parameter
- 170: size
- 172: distance
- 174: front camera
- 176: distance
- 178: input unit
- 180: input area
- 182: virtual keypad
- 184: button
- 186: microphone
- 188: eye movements
- 190: luminance sensor
- 192: storage unit
- 210: computer-implemented method for determining a visual performance of at least one eye of a person
- 212: identifying (step)
- 214: recognizing (step)
- 216: determining (step)
- 218: visual performance
- 220: refractive error
- 222: comparing (step)
- 224: generating (step)
- 226: initiating (step)
- 228: spectacle lens

## Claims

1. A computer-implemented method (210) for determining a visual performance (218) of at least one eye (112) of a person (114), the method (210) comprising the following steps:
a) identifying (212) a point in time indicating that a person (114) is able to perceive at least one visual target (128) from a reaction of the person (114);
b) determining (216) a visual performance (218) of at least one eye (112) of the person (114) by using at least one first piece of information about the at least one visual target (128) and at least one second piece of information about at least one parameter (168) related to the at least one visual target (128) at the point in time, **characterized by**
c) recognizing (214) the at least one visual target (128) and the at least one parameter (168) related to the at least one visual target (128) at the point in time by scanning a natural environment (122) of the person (114).

2. The computer-implemented method (210) according to the preceding claim, wherein the visual performance (218) is selected from at least one of
- a contrast sensitivity;
- a visual acuity;
- a color vision;
- a visual field.

3. The computer-implemented method (210) according to any one of the preceding claims, wherein recognizing (214) the at least one visual target comprises at least one of detecting at least one object (136) in the natural environment (122) of the person (114) or segmenting the at least one object (136) from the natural environment (122) of the person (114), and wherein recognizing (214) the at least one visual target (128) and the at least one parameter (168) related to the at least one visual target (128) comprises identifying (212) the at least one visual target (128) and determining the at least one parameter (168) related to the at least one visual target (128) from at least one scan of the at least one object (136).

4. The computer-implemented method (210) according to any one of the preceding claims, wherein recognizing (214) the at least one visual target (128) comprises classifying the at least one visual target (128) according to the at least one parameter (168) related to the at least one visual target (128).

5. The computer-implemented method (210) according to any one of the preceding claims, wherein the at least one visual target (128) is selected from at least one of a number (130); a letter (132); or a symbol (134), wherein the at least one parameter (168) related to the at least one visual target (128) is selected from at least one of
- a size (170);
- an angular size;
- a contrast;
- a contrast gradient;
- a color;
- a color gradient;
- a spatial position;
- a velocity of movement;
- a distance to the person (114);
of the at least one visual target (128).

6. The computer-implemented method (210) according to any one of the preceding claims, wherein identifying (212) the point in time indicating that the person (114) is able to perceive the at least one visual target (128) comprises recording the reaction of the person (114) to the at least one visual target (128), wherein the reaction of the person (114) to the at least one visual target (128) is selected from at least one of
- a touch response;
- a voice entry;
- gesture recognition;
- an eliciting of at least one type of eye movements in the at least one eye (112) of the person (114).

7. The computer-implemented method (210) according to any one of the preceding claims, further comprising a step of
d) repeating steps a) to c) by using at least one of a modified parameter (168) related to the at least one visual target (128) or a different visual target (128).

8. The computer-implemented method (210) according to any one of the preceding claims, wherein determining (216) the visual performance (218) of the at least one eye (112) of the person (114) comprises a step of
e) comparing (222) the at least one visual target (128) and the at least one parameter (168) related to the at least one visual target (128) as recognized by scanning the natural environment (122) of the person (114) with at least one further piece of information about the at least one visual target (128) at the point in time as determined from the reaction of the person (114).

9. The computer-implemented method (210) according to any one of the preceding claims, wherein determining (216) the visual performance (218) of the at least one eye (112) of the person (114) comprises a step of
f) detecting at least one irregular movement in the face of the person (114), wherein the at least one irregular movement indicates visual strain of the at least one eye (112) of the person (114), and classifying the at least one irregular movement using a score related to the visual performance (218) of the at least one eye (112) of the person (114).

10. The computer-implemented method (210) according to any one of the preceding claims, further comprising a step of
g) generating (224) at least one piece of output information to the person (114), wherein the at least one piece of output information is transmitted to the person (114) by using at least one of a voice output, a visual output, or a tactile output.

11. The computer-implemented method (210) according to any one of the preceding claims, further comprising at least one further step selected from at least one of h) measuring a distance between the at least one visual target (128) and the at least one eye (112) of the person (114);
i) measuring a luminance level of the at least one visual target (128), wherein the visual performance (218) of the at least one eye (112) of the person (114) is determined by using a measured value for the luminance level of the at least one visual target (128);
j) determining at least one refractive error (220) of the at least one eye (112) of the person (114) by using the visual performance (218) of the at least one eye (112) of the person (114);
k) initiating (226) a process for producing at least one spectacle lens (228) using the at least one refractive error (220) of the at least one eye (112) of the person (114).

12. A computer program comprising instructions which, when the program is executed by a computer, cause the computer to carry out the computer-implemented method (210) for determining a visual performance (218) of at least one eye (112) of the person (114), wherein the method (210) comprises the following steps:
a) identifying (212) a point in time indicating that a person (114) is able to perceive at least one visual target (128) from a reaction of the person (114);
b) determining (216) a visual performance (218) of at least one eye (112) of the person (114) by using at least one first piece of information about the at least one visual target (128) and at least one second piece of information about at least one parameter (168) related to the at least one visual target (128) at the point in time,
**characterized by**
c) recognizing (214) the at least one visual target (128) and the at least one parameter (168) related to the at least one visual target (128) at the point in time by scanning a natural environment (122) of the person (114).

13. A method for producing at least one spectacle lens (228), wherein producing the at least one spectacle lens (228) comprises processing at least one lens blank by using at least one centration parameter, wherein the at least one centration parameter is determined by using a computer-implemented method (210) for determining a visual performance (218) of at least one eye (112) of a person (114), the method (210) comprising the following steps:
a) identifying (212) a point in time indicating that a person (114) is able to perceive at least one visual target (128) from a reaction of the person (114);
b) determining (216) a visual performance (218) of at least one eye (112) of the person (114) by using at least one first piece of information about the at least one visual target (128) and at least one second piece of information about at least one parameter (168) related to the at least one visual target (128) at the point in time,
**characterized by**
c) recognizing (214) the at least one visual target (128) and the at least one parameter (168) related to the at least one visual target (128) at the point in time by scanning a natural environment (122) of the person (114).

14. A device (110) for determining a visual performance (218) of at least one eye (112) of a person (114), the device (110) comprising:
- at least one processing unit (166), wherein the at least one processing unit (166) is configured to
∘ identify a point in time indicating that a person (114) is able to perceive at least one visual target (128) from a reaction of the person (114); and ∘ determine a visual performance (218) of at least one eye (112) of the person (114) by using at least one first piece of information about the at least one visual target (128) and at least one second piece of information about at least one parameter (168) related to the at least one visual target (128) at the point in time,
**characterized by**
- at least one scanning unit (120), wherein the at least one scanning unit (120) is configured to scan a natural environment (122) of the person (114),
and that the at least one processing unit (166) is further configured to
∘ recognize the at least one visual target (128) and the at least one parameter (168) related to the at least one visual target (128) at the point in time by scanning a natural environment (122) of the person (114).

15. The device (110) according to the preceding claim, wherein the at least one at least one scanning unit (120) is selected from at least one of
- a camera (124, 174);
- a depth sensor;
- an acceleration and position sensor.
